Europäisches Patentamt

European Patent Office

Office européen des brevets

(11) Publication number: **0 311 604 B1**

# EUROPEAN PATENT SPECIFICATION

(45) Date of publication of patent specification: **25.03.92**

(51) Int. Cl.⁵: **G01N 33/543**, G01N 33/569, //B01D37/00

(21) Application number: **86904435.4**

(22) Date of filing: **26.06.86**

(86) International application number: **PCT/SE86/00309**

(87) International publication number: **WO 88/00345 (14.01.88 88/02)**

(54) **METHOD AND APPARATUS FOR OUALITATIVE AND/OR OUANTITATIVE ANALYSIS OF ANTIGENS, ANTIBODIES, MIRCROORGANISMS OR OTHER CELLS.**

(43) Date of publication of application:
**19.04.89 Bulletin 89/16**

(45) Publication of the grant of the patent:
**25.03.92 Bulletin 92/13**

(84) Designated Contracting States:
**DE FR GB IT NL**

(56) References cited:
EP-A- 141 547          WO-A-85/05451
WO-A-86/02160          FR-A- 2 514 367
GB-A- 2 084 317        SE-A-85 014 413
US-A- 4 407 943        US-A- 4 459 361

PATENT ABSTRACTS OF JAPAN, vol. 8, no. 82, 27 December 1983; p.268

CHEMICAL ABSTRACTS, vol. 89, 1978, Columbus, OH (US); no. 2665g

(73) Proprietor: **FASSL AB**
**Ole Römers väg 12**
**S-223 63 Lund(SE)**

(72) Inventor: **KVIST CHRISTENSEN, Karen**
**Genvägen 3**
**S-297 00 Degeberga(SE)**
Inventor: **CHRISTENSEN, Poul**
**Genvägen 3**
**S-297 00 Degeberga(SE)**

(74) Representative: **Wiklund, Ingrid Helena et al**
**AWAPATENT AB P.O. Box 5117**
**S-200 71 Malmö(SE)**

## Description

The present invention relates to a reagent kit and a method for qualitative and/or quantitative analysis of antigens, antibodies, microorganisms or other cells in a liquid specimen as well as an apparatus for performing the method.

Background of the invention

Laboratories working within microbiological, immunological or clinical chemistry etc. make extensive use of reactions between antigens and antibodies. Many complex molecules are antigenic, i.e. they give rise to the production of specific antibodies in man and animal. These antibodies are proteins which, with very few exceptions, react but with corresponding antigens or very closely related combinations. Antibodies play an important part in the protection against microorganisms. They are to be found in blood and tissue in high concentrations and are directed towards an overwhelming number of different antigens. The production of antibodies starts early in life and continues step by step as the body is exposed to the antigens. However, a few other mechanisms can also start the production of antibodies.

Because of their specific way of reacting, antibodies are used in many technical applications, e.g. demonstration and identification of bacteria, viruses and fungi. Furthermore they are used in methods for quantification of the body's own elements. The antibodies are often available in so-called antisera, i.e. sera (blood without cells) that have been taken from an animal or a human who has been exposed to the antigen that one wants to demonstrate. It is often necessary to "absorb" the antiserum in order to remove unwanted antibodies therefrom. Antibodies against other antigens can give rise to "false" reactions in a test system. It is now even possible to produce monoclonal antibodies by making use of modern gene technology. Monoclonal antibodies are antibodies produced by just one cell population which originates from one cell, a single-clone. Thus, these antibodies are antibodies of one type only and are not contaminated by other antibodies like in antisera.

Demonstration and quantification of antibodies are also of great medical importance. For instance, the presence of specific antibodies against a certain virus can indicate an infection in progress against this virus or the presence of immunity (ability to protect) against this microorganism.

Different ways of demonstrating antigen-antibody-interactions are previously known. A summary of the different methods presently used follows below.

Agglutination: Antibodies are bound to an antigen situated on particles, which results in a visible aggregation of the particles. The existence of aggregates can be seen with the naked eye or under microscope. This is a very quick method (it can be performed in a few minutes) which is readily carried through. The susceptibility to small quantities of substance is high, and there are few possibilities of false reactions. The disadvantages are, inter alia, that it is not possible to accomplish an exact quantitative analysis and that the interpretation is subjective.

Precipitation: Soluble antigens and antibodies interact and give rise to a "network", i.e. a large complex consisting of many subunits of antigen and antibody. The complex settles and is visible as a (usually) white precipitation. The precipitation can be demonstrated in a transparent tube or in a transparent firm gel. In the latter case, antibodies and antigens diffuse against each other from different positions in the gel or are forced to meet by the application of electric current. Precipitation can also be combined with labelled antibodies (see below) in order to increase susceptibility (the ability to demonstrate small quantities of antigens or antibodies).

This method is relatively simple but slow (takes at least 1 h). Quantitative analysis is possible, but the susceptibility to small quantities of substance is low. The interpretation is subjective or objective, and there are few possibilities of false reactions.

Methods using labelled antibodies: the fraction of antibodies in a serum can be purified and "labelled" with radioactivity (Radio Immuno Assay = RIA), fluorescing compounds or enzymes. The "labelling" is combined with the antibody by means of a chemical reaction. Radioactivity and fluorescence are demonstrated by the use of specific equipment suitable for such purposes. The presence of enzyme-labelled antibodies is shown by addition of a substrate, i.e. a substance which is decomposed by the enzyme and, as a result, causes a change of colour. The change of colour can be shown quantitatively by using a spectro-photometer. This method is called ELISA (Enzyme-Linked-Immuno-Sorbent-Assay).

The advantage of ELISA is that it makes an exact quantitative analysis possible and, moreover, it has a very high susceptibility to small quantities of substance. The interpretation of the results is objective. There are, however, several disadvantages. Thus, the reaction is slow (at least 1 h), and the method is complicated and causes false reactions.

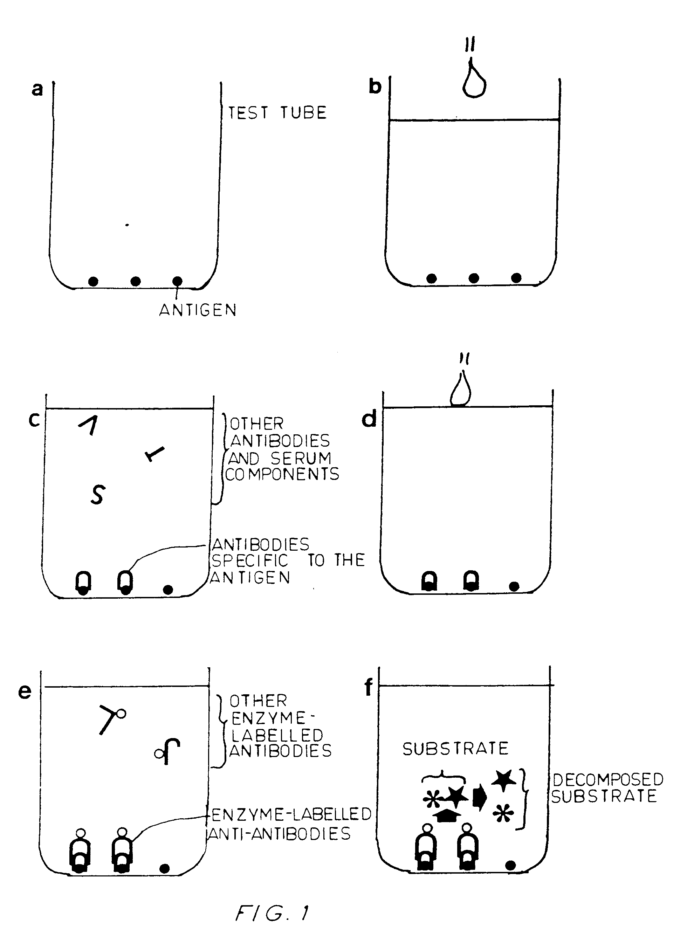

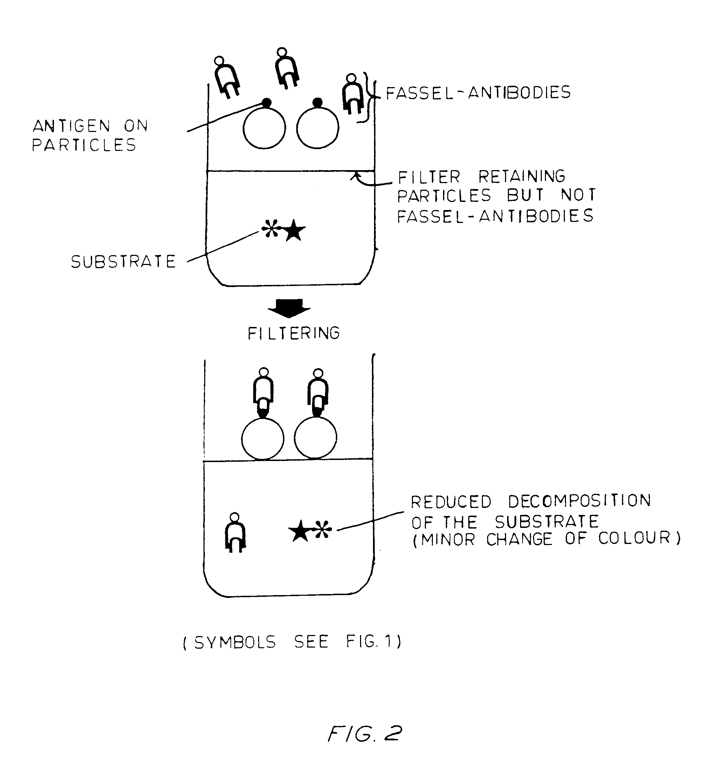

The ELISA method is often used for quantification of antibodies in accordance with the following process chart (see Fig. 1):

a) the antigen is bound to the inner wall of a test tube;

b) the liquid (usually serum) to be tested in regard to antibodies is added;

c) antibodies that are not bound and other serum components are removed by washing the test tube several times with e.g. a saline solution;

d) enzyme-labelled antibodies from an animal that has been exposed to human antibodies are added (anti-antibodies);

e) enzyme-labelled antibodies which do not react are removed by washing;

f) finally the substrate is added, and the change of colour is gauged by means of a spectro-photometer.

It thus is clear that this method necessitates several steps of pipetting. For this reason, a great deal of different machinery for automation of this method is available on the market. Devices have been designed for the washing processes, for the simultaneous "reading" of a large number of test tubes in the spectro-photometer and for the steps of pipetting and diluting. All products are expensive and adapted for the simultaneous testing of many specimens. To date, no device has been designed for the automatic testing of single-specimens.

If, instead, a known number of antibodies is used in the test system, this method can even be used for the quantification of antigens.

Labelled antigens: Instead of labelling the antibodies, the antigens can be labelled with radioactivity (Radio Immuno Assay = RIA), fluorescent compounds or enzymes. The basic method is mainly the same as has been described for the methods using labelled antibodies.

Other methods are also known which can be practised in a few specialised tests only.

To sum up, it may be mentioned that "test sets" based on demonstration of agglutination, precipitation or the ELISA technique are avabilable on the market. Also other test sets not using the above methods are available, but in general they are applicable to a very limited extent.

Especially the ELISA system is used to a large extent because of its high susceptibility, i.e. its ability to demonstrate small quantities of substance. However, these tests can only be used in large laboratories since only specially trained staff can handle them.

When qualified staff is not available, agglutination and precipitation are frequently used when there is no demand for extreme accuracy. These methods are more easily performed. Particularly when reactions of agglutination were concerned, it has been possible to decentralise the use to small clinical units.

Advantages and disadvantages of the different methods are summed up in Table 1 below.

Table 1

| Advantages and disadvantages of prior art methods for demonstrating reactions between antigens and antibodies | | | | | | |
|---|---|---|---|---|---|---|
| Method | Speed of the method | Performance | Exact quantification possible? | Susceptibility to small quantities | Interpretation of the test result | Possibilities of false reactions |
| Agglutination | very fast (within a few minutes) | simple | no | high | subjective | few |
| Precipitation | slow (at least 1 h) | relatively simple | yes | low | subjective or objective | few |
| ELISA | slow (at least 1 h) | complicated | yes | very high | objective | several |
| Immuno-fluorescence | rather slow (at least 30 min) | relatively simple | generally yes | high | subjective or objective | a great deal |
| Radio Immuno Assay (RIA) | slow (at least 1 h) | complicated | yes | very high | objective | a great deal |

EP 0 311 604 B1

It is an object of the present invention to provide a reagent kit, a method and an apparatus for qualitative and/or quantitative analysis of an analyte consisting of antigens, antibodies, microorganisms or other cells in a liquid specimen. According to the invention, a quick and very susceptive analysis is achieved, which can be performed by persons without special training.

Earlier attempts to introduce simple-to-use-and-interpret analysis techniques for decentralised use have not included ideas such as the present invention. One example is US Patent 4,459,361 where particle aggregation is formed in the presence of an analyte. Aggregated particles will not pass through a filter. The amount of analyte in the sample is estimated by conventional methods from the amount of aggregated particles on the filter or unaggregated particles which pass through the filter.

A similar idea is presented in PCT Application WO-86/02160 where particles like bacteria, viruses and pollen are detected by trapping in a device consisting of a number of filters with successively decreasing permeability. The particles are numbered and identified by size and by the addition of labelled specific antibodies.

EP-A-141,547 describes an assay construction where the analyte is detected by a binding partner, such as an antibody, and a labelled component. The "label such as an enzyme ... can be bonded to another compound which provides the binding capability to link the label to the component". The analyte, binding partner and labelled component complex is separated from the non-reacted binding partner and labelled component by a separation means. The presence and quantity of analyte are then determined by e.g. an enzyme substrate reaction.

GB-A-2,084,317 relates to a method where the analyte to be determined competes with a known amount of analyte covalently bound to a reactant for an analyte specific antibody. A second enzyme-labelled antibody recognises the first analyte-specific antibody. The complex is visualised by an enzyme substrate reaction. The analyte to be determined and the reactant-bound analyte are separated by an immunosorbent carrying antibodies to the reactant.

FR-A-2,514,367 describes a procedure to detect pathogenic microorganisms by performing an affinity chromatography.

The method according to the present invention is characterised by bringing the specimen into contact with a solution containing a certain quantity of a preprepared immuno-complex of analyte-specific antibodies and enzyme-labelled antibodies against immunoglobulins (FASSEL-antibodies), and, except when the analyte consists of large microorganisms or cells, analyte-specific binding, inert particles, whereby a further complex is formed between the analyte and said immuno-complex of the analyte-specific antibodies and the enzyme-labelled antibodies and, optionally, the analyte-specific binding inert particles, whereupon the solution is made to pass through a filter that retains the complex-bound particles and the large microorganisms or cells but lets the unlinked immuno-complex of analyte-specific antibodies and enzyme-labelled anti-antibodies pass, which subsequently are demonstrated in that the solution is brought into contact with a specific substrate that is decomposed by the enzyme of the FASSEL-antibodies into products demonstrable by colour.

The term "analyte-specific binding, inert particle" or "inert particle" as used herein means an analyte-specific binding particle/macromolecule inert to all other reactions but the specific binding.

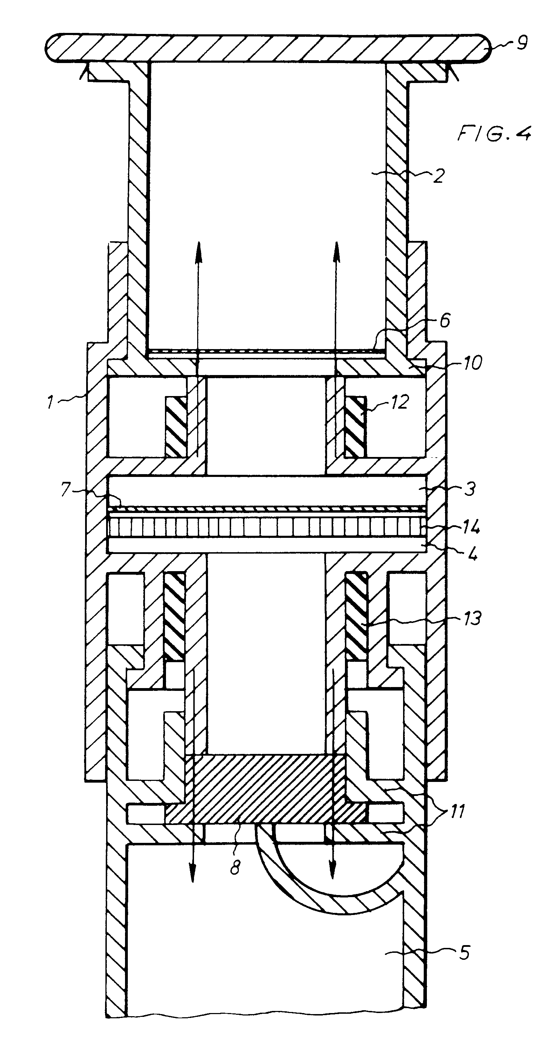

The invention will be described more closely below with reference to the accompanying drawings in which Fig. 1 shows the previously known ELISA method, from which the present invention proceeds; Fig. 2 shows schematically the inventive method; Fig. 3 shows the production of the immuno-complex of analyte-specific antibodies and enzyme-labelled antibodies against immunoglobulins, or the so called specific, enzyme-labelled anti-antibodies (or FASSEL-antibodies) as used according to the invention, and Fig. 4 shows an embodiment of the apparatus according to the invention.

Thus, the invention also relates to an apparatus for performing the method. The apparatus is characterised in that it comprises a tubular container 1 with a lid, which is divided into an upper chamber 2, a filter chamber 3, 4 and a lower chamber 5, that the upper chamber 2 is separated from the filter chamber 3, 4 by a membrane 6, that a filter 7, such as a sterile filter, is fitted in the filter chamber 3, 4, and that the filter chamber 3, 4 is separated from the lower chamber 5 by a detachably fitted closure device 8.

Furthermore, the apparatus comprises a locking member 10 for the upper chamber 2 and a locking member 11 for the closure device 8 as well as packing rings 12, 13 so as to maintain a subpressure in the container 1. The locking members 10 and 11, respectively, can be operated mechanically. The filter 7 is preferably supported by a filter support 14.

The method according to the present invention is called FASSEL which is an abbreviation of Filtration of Antibody, Separated, Specific and Labelled.

The principle of the method according to the invention is shown schematically in Fig. 2 with demonstration of antigens as an example of the practice. Antigens generally being very small particles, are first bound to larger, inert particles. Subsequently they are brought into contact with a certain quantity of specific, enzyme-labelled anti-antibodies (FASSEL-antibodies). The solution is then caused to pass through a filter with a pore size such as retains the particles with the complex-bound FASSEL-antibodies. The FASSEL-antibodies that are not complex-bound pass through the filter and down into a substrate solution where the enzyme decomposes the substrate while producing a change of colour. This change of colour can be read visually or by a spectro-photometer. By comparing it to calibrated colour charts, one can read the quantity of FASSEL-antibodies that has passed through the filter. Thus, by adding a certain quantity of FASSEL-antibodies at the beginning, one can demonstrate on the one hand the presence of a specific antigen in the specimen and, on the other hand, the quantity thereof.

The complex of analyte-specific antibodies and enzyme-labelled antibodies against immunoglobulins, used in the present invention, are produced in the following way: One adopts well-known principles of so-called chromatography of affinity. The intention is to separate the specific antibodies to be used in the method according to the invention, i.e. to remove all other antibodies and serum components from the specific antibodies. Furthermore, the specific antibodies are to be labelled with enzyme, which is achieved by application of enzyme-labelled anti-antibodies. Enzyme-labelled substances that are not bound to the specific antibodies are removed. All these steps are performed in one process, which is a completely new principle that has not previously been used within the chromatography of affinity.

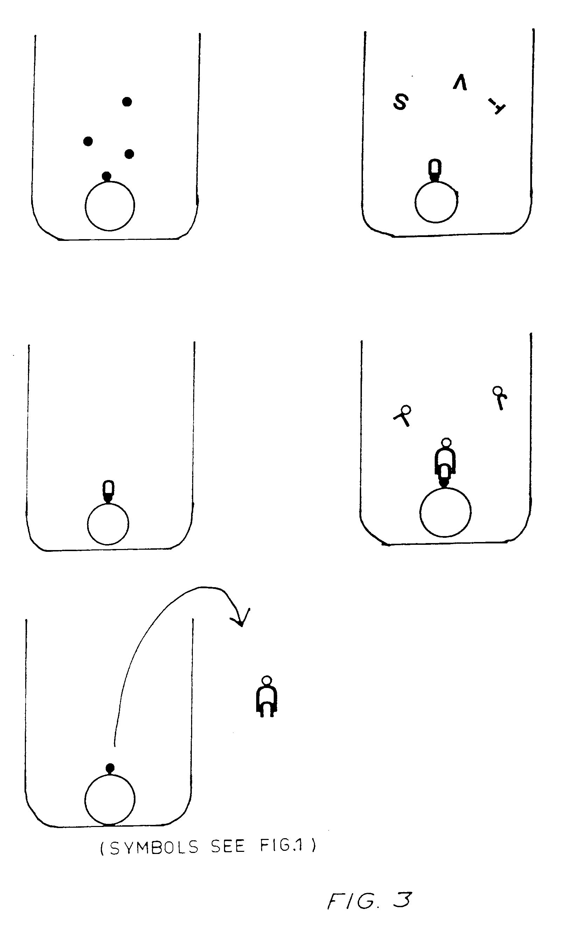

FASSEL-antibodies against soluble antigens are produced in accordance with the following method (see Fig. 3):

a) The antigen is bound to a carbohydrate polymer, a latex particle or another particle by per se known methods.

b) The antiserum from which the specific antibodies are to be removed, is mixed with the particles.

c) The particles are washed free from unlinked serum components by the use of a neutral hypertonic or slightly acid isotonic solution so as to remove specific antibodies with a low affinity to the antigen (buffer example: 2.5 M NaCl, 0.03 M phosphate buffer, pH 7.2 or 0.15 M NaCl, 0.03 M phosphate buffer, pH 5).

d) Enzyme-labelled anti-immunoglobulin (from animal or man, against animal or human immunoglobulin) is added, and unlinked enzyme-labelled anti-immunoglobulin is washed off.

e) The antibodies bound to the particles are eluated by means of a solution at low pH (e.g. 2.5) or by 3 M KSCN at neutral pH.

f) The particles are removed by centrifugation or filtration. The solution of FASSEL-antibodies is neutralised or dialysated against a buffer so as to re-establish the reactivity of the antibodies.

The FASSEL-antibodies are thus ready preprepared analyte-specific enzyme-labelled immuno-complexes that are to be brought into contact with the specimen.

Horseradish peroxidase is referred to as an example of enzymes which can be used for labelling the anti-immunoglobulin. Many different preparations of enzyme-labelled anti-immunoglobulin are generally available.

FASSEL-antibodies against microorganisms, such as bacteria or fungi or other cells, can be produced by using the intact microorganisms or cells instead of using inert particles bearing purified antigens.

The principle of the present invention can be applied to one antigen-antibody system only, a so-called single-FASSEL, or in connection with several FASSEL systems, so-called multi-FASSEL systems. In a single-FASSEL, only one antigen-antibody system is utilised. In multi-FASSEL, the specimen is fitted in a place that divides into two or more lines, each connected with a FASSEL system of its own. Multi-FASSEL makes it possible to analyze a specimen in regard to two or more parameters.

A closer description of an embodiment of the apparatus according to the invention follows below. The apparatus comprises a tubular container 1 provided with a lid 9. The container is divided into three chambers. The upper chamber 2 contains the FASSEL-antibodies (and when required, a disinfectant), the intermediate chamber (the filter chamber) 3, 4 contains a filter 7, and the lower chamber 5 contains the substrate. The FASSEL antibodies in the upper chamber 2 are first separated from the filter chamber 3, 4 by means of a membrane 6. A closure device 8 is detachably fitted between the filter chamber 3, 4 and the lower chamber 5. The membrane 6 and the closure device 8 can be destroyed or removed mechanically when carrying out the method. The container is sealed by means of suitable packing members. The lower chamber 5 contains a specific substrate that can be decomposed by the enzyme of the FASSEL-antibodies in the upper chamber 2.

The sealing between the chambers is broken by producing a positive pressure in the upper chamber 2 or a negative pressure (subpressure) in the lower chamber 5. The positive pressure in the upper chamber 2 can be obtained e.g. by forcing a cylinder in a downward direction in the chamber. The negative pressure can be established in the system by producing a subpressure in the lower chamber 5.

The solution with the analyte is introduced in the upper chamber 2, where the FASSEL-antibodies specific to the analyte are bound as a complex to the analyte which in the present case is bound to inert particles of such a size that they cannot pass through the filter 7 in the filter chamber 3, 4. Subsequently, the membrane 6 is destroyed, e.g. by outside pressing of the container 1 which preferably is made of a slightly flexible material. The solution is then forced through the filter 7 either by a pressure produced above the filter or by a subpressure established in the lower chamber 5. Only those FASSEL-antibodies that are not complexbound, are able to pass through the filter, and as a result they come into contact with the substrate in the lower chamber 5. The enzyme of the FASSEL-antibodies decompose the specific substrate into coloured products which are readable visually or spectro-photometrically.

The container with the specific FASSEL antibodies is preferably provided with information on the interpretation and the purpose of the test.

In a completely manual analysis according to the present invention, the decomposition of the substrate is demonstrated as a change of colour visible with the naked eye, and this change of colour is compared to a standard colour chart.

A computer ("Computer-FASSEL") can also be used for interpreting the change of colour via a spectro-photometer. The limits of the colour shades for the interpretation, which are unique for each batch of FASSEL-tubes, are on the tube for the computer to read.

The specimen to be tested is introduced into the upper chamber of a FASSEL-container which is placed in an application space in the computer. The computer preferably is programmed to ask for the patient's identification data. When the identification is completed, the computer accepts the specimen and states the patient's data, the type of test and the necessary time to perform the test. After a certain incubation (time and temperature are specific to each test), the machine neutralises the sealing between the chambers. After further incubation (which is also specific to each test), the computer interprets the results in relation to the limits of positive and negative results or the quantitative formula stated on the test container. Further information which the computer can provide are the limits of reliable test results, advice on complementary laboratory tests in relation to the obtained result or clinical background information.

Each such "Computer-FASSEL" can interpret all different FASSEL-tubes. The computer is designed to accept the tests one by one, independently of the purpose of the test.

The spectro-photometer is calibrated by means of two standard colour tubes (one for "high value" and one for "low value"). The test tubes are labelled with information on the calibration. It is also possible to connect a printer with the computer and to connect the computer with a central laboratory for exchange of current information.

Some examples of the application of the method according to the invention follow below. The examples are not meant to be restrictive.

EXAMPLE 1

Qualitative and quantitative analysis of bacteria, fungi or other cells (e.g. lymphocytes)

The specimen to be tested is mixed with the preprepared immuno-complex of analyte-specific antibodies and enzyme-labelled antibodies against immunoglobulins in the upper chamber of an apparatus according to the invention. If bacteria, fungi or cells that react on the preprepared immuno-complex are present, the FASSEL-antibodies will be retained in the upper chamber, when a difference in pressure is produced above the filter. Thus, this filter has such small pores that the bacteria, fungi or other cells cannot pass therethrough.

To avoid unspecific reactions from bacteria reacting on antibodies independent of their specificity, normal serum may be added for blockage.

Subsequently the unlinked FASSEL-antibodies decompose the substrate in the lower chamber of the apparatus. The decomposition is seen as a change of colour. By using a predetermined quantity of the preprepared immuno-complex, the change of colour in the lower chamber will also be a gauge of the quantity of bacteria, fungi or other cells present in the specimen.

EXAMPLE 2

Qualitative and quantitative analysis of viruses

In general, viruses are far too small to be retained by filters suitable for this invention. Therefore, the following modification is used. The upper chamber contains inert particles linked to suitable antivirus-antibodies, as well as a complex of virus specific antibodies and enzyme-labelled anti-antibodies. When the virus is compounded with these reagents, it will be bound to the particles, and then the particles will be covered by the complex of virus-specific antibodies and enzyme-labelled antibodies reacting on the virus. These complexes of virus, inert particle and complex of virus-specific antibody and enzyme-labelled anti-antibody will be retained by the filter.

Like in Example 1, a quantification of the virus can also be obtained here by reading the intensity of the colour of the substrate solution in the lower chamber.

However, this system can give rise to falsely positive reactions if unusually high levels of viruses are present in the specimen. If a computer is used, it may be programmed to advise the user to test also a diluted specimen so as to obtain a negative result.

## EXAMPLE 3

### Qualitative and quantitative analysis of soluble antigens

These antigens can be structures present in body fluid or tissue or in the environment. Examples of antigens are serum components which usually are demonstrated by clinical chemistry, hormones and soluble antigens to microorganisms including hepatit- and HIV-virus. This test is performed mainly in the same way as in Example 2.

## EXAMPLE 4

### Qualitative and quantitative analysis of antibodies

In order to analyse antibodies in a specimen, the specimen is compounded on the one hand with inert particles to which antigens corresponding to the antibodies to be demonstrated have been bound and, on the other hand, with a complex of antibodies specific to the antibodies that are to be demonstrated and enzyme-labelled anti-antibodies. Antibodies present in the specimen will then be bound to the antigens on the particles, whereupon the said complex is, in turn, bound to the antibodies in the specimen. A quantity of the FASSEL-antibodies corresponding to the present quantity of antibodies in the specimen will thus be retained by the filter, while the remaining part of the FASSEL-antibodies pass through the filter and react on the substrate.

The reagent kit, method and the apparatus according to the invention combine the high susceptibility of the ELISA-system with operations that are very simple from a technical point of view and eliminate the many possibilities of false reactions, which are serious disadvantages of the well-known ELISA-system.

Moreover, the possibility of connecting the method and the apparatus according to the invention to a computer brings many advantages over prior art methods:

(a) The "Computer-FASSEL" is one apparatus for performing a great number of different tests which are now used in many different laboratories and within different medical disciplines.

(b) The test system is fully automated. Any person who can collect a specimen can perform a test in accordance with the invention, without special training or special manuals.

(c) The method and the apparatus according to the invention mean decentralization, which implies that tests which to date have been very intricate can be performed in small laboratory units from now on.

(d) Results are obtained quickly, even concerning highly specialised tests, and they are already available while the patient still is in the laboratory.

(e) In addition to the test results, the computer can even be programmed to provide important background information, e.g. the current epidemiological situation in the community.

When not connected to a computer, the method according to the invention (the so-called "Colour-FASSEL") can be performed and interpreted very easily and can therefore be performed by anybody who can collect a specimen and read a short instruction on the outside of a test tube. The test can even be performed in the patient's home under the most primitive circumstances with no equipment other than the test tube according to the invention. Some of the advantages of this method with direct reading with the naked eye are as follows:

(a) it is quick;

(b) it requires very little work and

(c) it can be applied to testing one single specimen in several different antigen-antibody systems at the same time.

8

**Claims**

1. A reagent kit for qualitative and/or quantitative analysis of an analyte consisting of antigens, antibodies, microorganisms or other cells, in a liquid specimen, **characterised** in that it comprises a preprepared immuno-complex of analyte-specific antibodies and enzyme-labelled antibodies against immunoglobulins, and a specific substrate that can be decomposed by the enzyme of the enzyme-labelled antibodies.

2. A method for qualitative and/or quantitative analysis of an analyte consisting of antigens, antibodies, microorganisms or other cells, in a liquid specimen, **characterised** in that the specimen is brought into contact with a solution containing a certain amount of a preprepared immuno-complex of analyte-specific antibodies and enzyme-labelled antibodies against immunoglobulins, and, except when the analyte consists of large microorganisms or cells, analyte-specific binding, inert particles, whereby a further complex is formed between the analyte and said immuno-complex of the analyte-specific antibodies and the enzyme-labelled anti-antibodies and, optionally, the analyte-specific binding inert particles, whereupon the solution is made to pass through a filter that retains the complex-bound particles and large microorganisms or cells but lets the unlinked immuno-complex of analyte-specific antibodies and enzyme-labelled anti-antibodies pass, which subsequently are demonstrated in that the solution is brought into contact with a specific substrate that is decomposed by the enzyme into products demonstrable by colour.

3. A method as claimed in claim 2, **characterised** in that the products demonstrable by colour are read visually or spectro-photometrically.

4. A method as claimed in claim 2 or 3, **characterised** in that, when the analyte consists of antibodies, the antibodies are bound as a complex on the one hand to said particles to which antigens have been bound that are specific to the antibodies to be analysed, and, on the other hand, to a complex of antibodies specific to said antibodies and enzym-labelled antibodies against immunoglobulins.

5. A method as claimed in claim 2 or 3, **characterised** in that, when the analyte consists of antigens or small microorganisms such as viruses, the antigens or viruses are bound as a complex on the one hand to particles to which antibodies have been bound that are specific to the antigens or viruses to be analysed and, on the other hand, to a complex of antibodies specific to said antigens and viruses, respectively, and enzyme-labelled antibodies against immunoglobulins.

6. An apparatus for performing qualititive and/or quantitative analysis of an analyte consisting of antigens, antibodies, microorganisms or other cells in a liquid specimen, **characterised** in that it comprises a tubular container (1) with a lid, which is divided into an upper chamber (2), a filter chamber (3, 4) and a lower chamber (5), in that the upper chamber (2) is separated from the filter chamber (3, 4) by a membrane (6), in that a filter (7), such as a sterile filter, is fitted in the filter chamber (3, 4), in that the filter chamber (3, 4) is separated from the lower chamber (5) by means of a detachably fitted closure device (8), in that the upper chamber (2) contains a solution containing a prepared immuno-complex of analyte-specific antibodies and enzyme-labelled antibodies against immunoglobulins, and, except when microorganisms or other cells are analysed, analyte-specific binding, inert particles to which antibodies and antigens, respectively, specific to the antigens and antibodies, respectively, have been bound, and in that the lower chamber (5) contains a specific substrate that can be decomposed by said complex.

7. An apparatus as claimed in claim 6, **characterised** in that a subpressure is maintained in the lower chamber (5).

8. An apparatus as claimed in any one of claims 6-7, **characterised** in that an optionally automatic spectro-photometer is arranged in connection with the lower chamber (5) for reading the colour reaction in the substrate.

**Revendications**

1. Trousse de réactifs destinée à l'analyse qualitative et/ou quantitative d'une substance à analyser constituée par des antigènes, des anticorps, des micro-organismes ou d'autres cellules, dans un échantillon liquide, caractérisé en ce qu'il comprend un complexe immun préparé à l'avance d'anticorps spécifiques de la substance à analyser et d'anticorps anti-immunoglobulines marqués par une enzyme, et un substrat spécifique décomposable par l'enzyme des anticorps marqués par une enzyme.

2. Procédé destiné à l'analyse qualitative et/ou quantitative d'une substance à analyser constituée par des antigènes, des anticorps, des micro-organismes ou d'autres cellules, dans un échantillon liquide, caractérisé en ce que l'échantillon est mis en contact avec une solution contenant une certaine quantité d'un complexe immun préparé à l'avance d'anticorps spécifiques de la substance à analyser et d'anticorps anti-immunoglobulines marqués par une enzyme et, sauf lorsque la substance à analyser est constituée par de gros micro-organismes ou de grosses cellules, de particules inertes fixant spécifiquement la substance à analyser, un autre complexe étant formé entre la substance à analyser et ledit complexe immun d'anticorps spécifiques de la substance à analyser et d'anticorps anti-anticorps marqués par une enzyme et, facultativement, de particules inertes fixant spécifiquement la substance à analyser, après quoi on fait passer la solution sur un filtre qui retient les particules liées au complexe et les gros micro-organismes ou les grosses cellules, mais laisse passer le complexe immun non lié d'anticorps spécifiques de la substance à analyser et d'anticorps anti-anticorps marqués par une enzyme, lesquels sont ensuite mis en évidence par mise en contact de la solution avec un substrat spécifique qui est décomposé par l'enzyme en produits identifiables par leur couleur.

3. Procédé selon la revendication 2, caractérisé en ce que les produits identifiables par leur couleur sont lus visuellement ou par spectrophotométrie.

4. Procédé selon la revendication 2 ou 3, caractérisé en ce que, lorsque la substance à analyser est constituée par des anticorps, les anticorps sont liés sous la forme d'un complexe d'une part auxdites particules auxquelles ont été fixés des antigènes spécifiques des anticorps à analyser et d'autre part à un complexe d'anticorps spécifiques desdits anticorps et d'anticorps anti-immunoglobulines marqués par une enzyme.

5. Procédé selon la revendication 2 ou 3, caractérisé en ce que, lorsque la substance à analyser est constituée par des antigènes ou de petits micro-organismes tels que des virus, les antigènes ou les virus sont fixés sous la forme d'un complexe d'une part aux particules auxquelles ont été fixés des anticorps spécifiques des antigènes ou des virus à analyser et, d'autre part, à un complexe d'anticorps spécifiques desdits antigènes et virus, respectivement, et d'anticorps anti-immunoglobulines marqués par une enzyme.

6. Appareil destiné à la mise en oeuvre de l'analyse qualitative et/ou quantitative d'une substance à analyser constituée par des antigènes, des anticorps, des micro-organismes ou d'autres cellules dans un échantillon liquide, caractérisé en ce qu'il comprend un conteneur tubulaire (1) à couvercle, divisé en une chambre supérieure (2), une chambre de filtration (3, 4) et une chambre inférieure (5), en ce que la chambre supérieure (2) est séparée de la chambre de filtration (3, 4) par une membrane (6), en ce qu'un filtre (7), un filtre stérile par exemple, est logé dans la chambre de filtration (3, 4), en ce que la chambre de filtration (3, 4) est séparée de la chambre inférieure (5) au moyen d'un dispositif de fermeture (8) amovible, en ce que la chambre supérieure (2) contient une solution contenant un complexe immun préparé à l'avance d'anticorps spécifiques de la substance à analyser et d'anticorps anti-immunoglobulines marqués par une enzyme et, sauf lorsqu'on analyse des micro-organismes ou d'autres cellules, de particules inertes fixant spécifiquement la substance à analyser auxquelles ont été fixés respectivement des anticorps et des antigènes spécifiques respectivement des antigènes et des anticorps et en ce que la chambre inférieure (5) contient un substrat spécifique décomposable par ledit complexe.

7. Appareil selon la revendication 6, caractérisé en ce qu'une pression négative est maintenue dans la chambre inférieure (5).

8. Appareil selon l'une quelconque des revendications 6 et 7, caractérisé en ce qu'un spectrophotomètre facultativement automatique est relié à la chambre inférieure (5) pour la lecture de la réaction colorée dans le substrat.

**Patentansprüche**

1. Reagenssatz zur qualitativen und/oder quantitativen Analyse eines aus Antigenen, Antikörpern, Mikroorganismen oder sonstigen Zellen bestehenden Analyten in einer flüssigen Probe, **gekennzeichnet** durch einen im voraus hergestellten Immunkomplex aus für den Analyten spezifischen Antikörpern und enzymmarkierten Antikörpern gegen Immunglobuline und ein spezifisches Substrat, das vom Enzym der enzymmarkierten Antikörper zersetzt werden kann.

2. Verfahren zur qualitativen und/oder quantitativen Analyse eines aus Antigenen, Antikörpern, Mikroorganismen oder sonstigen Zellen bestehenden Analyten in einer flüssigen Probe, dadurch **gekennzeichnet**, dass die Probe mit einer Lösung in Kontakt gebracht wird, enthaltend eine gewisse Menge eines im voraus hergestellten Immunkomplexes aus für den Analyten spezifischen Antikörpern und enzymmarkierten Antikörpern gegen Immunglobuline und, ausser wenn der Analyt aus grossen Mikroorganismen oder Zellen besteht, aus für den Analyten spezifischen, bindenden, inerten Teilchen, wodurch ein weiterer Komplex zwischen dem Analyten und dem Immunkomplex aus den für den Analyten spezifischen Antikörpern und den enzymmarkierten Antiantikörpern und eventuell den für den Analyten spezifischen, bindenden, inerten Teilchen gebildet wird, wonach die Lösung durch ein Filter geleitet wird, das die komplexgebundenen Teilchen und grosse Mikroorganismen oder Zellen zurückhält, aber den nichtgebundenen Immunkomplex aus für den Analyten spezifischen Antikörpern und enzymmarkierten Antiantikörpern durchlässt, welche dann dadurch nachgewiesen werden, dass die Lösung mit einem spezifischen Substrat in Kontakt gebracht wird, das vom Enzym in farbenmässig nachweisbare Produkte zersetzt wird.

3. Verfahren nach Anspruch 2, dadurch **gekennzeichnet**, dass die farbenmässig nachweisbaren Produkte visuell oder spektrophotometrisch gelesen werden.

4. Verfahren nach Anspruch 2 oder 3, dadurch **gekennzeichnet**, dass, wenn der Analyt aus Antikörpern besteht, die Antikörper teils an den genannten Teilchen, an welchen für die zu analysierenden Antikörper spezifische Antigene gebunden worden sind, teils an einem Komplex aus Antikörpern, die für die genannten Antikörper und enzymmarkierten Antikörper gegen Immunglobuline spezifisch sind, als ein Komplex gebunden werden.

5. Verfahren nach Anspruch 2 oder 3, dadurch **gekennzeichnet**, dass, wenn der Analyt aus Antigenen oder kleinen Mikroorganismen, z.B. Viren, besteht, die Antigene oder Viren teils an Teilchen, an welchen Antikörper gebunden worden sind, die für die zu analysierenden Antigene oder Viren spezifisch sind, teils an einem Komplex aus Antikörpern, die für die Antigene bzw. Viren spezifisch sind, und enzymmarkierten Antikörpern gegen Immunglobuline als ein Komplex gebunden sind.

6. Vorrichtung zum Durchführen einer qualitativen und/oder quantitativen Analyse eines Analyten aus Antigenen, Antikörpern, Mikroorganismen oder sonstigen Zellen in einer flüssigen Probe, dadurch **gekennzeichnet**, dass sie einen rohrförmigen, mit einem Deckel versehenen Behälter (1) umfasst, der in eine obere Kammer (2), eine Filterkammer (3, 4) und eine untere Kammer (5) aufgeteilt ist, dass die obere Kammer (2) mittels einer Membran (6) von der Filterkammer (3, 4) getrennt ist, dass ein Filter (7), beispielsweise ein Sterilfilter, in der Filterkammer (3, 4) angebracht ist, dass die Filterkammer (3, 4) mittels eines lösbar angeordneten Verschlusses (8) von der unteren Kammer (5) getrennt ist, dass die obere Kammer (2) eine Lösung enthält, enthaltend einen im voraus hergestellten Immunkomplex aus für den Analyten spezifischen Antikörpern und enzymmarkierten Antikörpern gegen Immunglobuline und, ausser wenn Mikroorganismen oder sonstige Zellen analysiert werden, aus für den Analyten spezifischen, bindenden, inerten Teilchen, an denen Antikörper bzw. Antigene, die für die Antigene bzw. Antikörper spezifisch sind, gebunden worden sind, und dass die untere Kammer (5) ein spezifisches Substrat enthält, das vom genannten Komplex zersetzt werden kann.

7. Vorrichtung nach Anspruch 6, dadurch **gekennzeichnet**, dass in der unteren Kammer (5) ein Unterdruck aufrechterhalten ist.

8. Vorrichtung nach einem der Ansprüche 6-7, dadurch **gekennzeichnet**, dass ein ev. automatischer Spektrophotometer zum Lesen der Farbenreaktion im Substrat in Verbindung mit der unteren Kammer (5) angeordnet ist.

**a**

TEST TUBE

ANTIGEN

**b**

**c**

OTHER ANTIBODIES AND SERUM COMPONENTS

ANTIBODIES SPECIFIC TO THE ANTIGEN

**d**

**e**

OTHER ENZYME-LABELLED ANTIBODIES

ENZYME-LABELLED ANTI-ANTIBODIES

**f**

SUBSTRATE

DECOMPOSED SUBSTRATE

*FIG. 1*

ANTIGEN ON PARTICLES

FASSEL-ANTIBODIES

FILTER RETAINING PARTICLES BUT NOT FASSEL-ANTIBODIES

SUBSTRATE

FILTERING

REDUCED DECOMPOSITION OF THE SUBSTRATE (MINOR CHANGE OF COLOUR)

(SYMBOLS SEE FIG.1)

FIG. 2

(SYMBOLS SEE FIG.1)

FIG. 3

FIG.4